# EUROPEAN PATENT SPECIFICATION

(11) **EP 3 074 006 B1**
(45) Date of publication and mention of the grant of the patent: **01.04.2020**
(21) Application number: 14808684.6
(22) Date of filing: 28.11.2014
(51) Int. Cl.: A61K 31/381, A61K 31/404, A61K 31/405, A61K 31/41, A61P 31/04, A61P 43/00

(54) **ZAFIRLUKAST AND ITS METABOLITES FOR USE AS ANTIBACTERIAL AGENTS**
ZAFIRLUKAST SOWIE DESSEN METABOLITEN ZUR VERWENDUNG ALS ANTIBAKTERIELLE WIRKSTOFFE
ZAFIRLUKAST ET SES MÉTABOLITES DESTINÉES A L'UTILISATION EN TANT QU'AGENTS ANTIBACTERIENS

(30) Priority: 29.11.2013 GB 201321089
(43) Date of publication of application: 05.10.2016
(73) Proprietor: University of East London, London E16 2RD (GB)
(72) Inventor: GEORGE, John Terry, London E16 2RD (GB); EMIOLA, Akintunde, London E16 2RD (GB)
(74) Representative: Leonard, Thomas Charles
(86) International application number: PCT/GB2014/053541
(87) International publication number: WO 2015/079254

(56) References cited:
- WO-A1-2009/152454
- WO-A1-2013/055674
- WO-A1-2014/068320
- RONALD N GOLDBERG ET AL: "Effects of a Leukotriene Antagonist on the Early Hemodynamic Manifestations of Group B Streptococcal Sepsis in Piglets", PEDIATRIC RESEARCH, vol. 20, no. 10, 1 October 1986 (1986-10-01), pages 1004-1008, XP055170907, ISSN: 0031-3998, DOI: 10.1203/00006450-198610000-00023
- SCHREIBER ET AL.: "Hemodynamic effects of heat-killed Group B beta- hemolytic Streptococcus in newborn lambs: Role of leukotriene D4", PEDIATRIC RESEARCH, vol. 31, no. 2, 1992, pages 121-126, XP55170914,
- KHOURY P ET AL: "Effect of montelukast on bacterial sinusitis in allergic mice", ANNALS OF ALLERGY, ASTHMA & IMMUNOLOGY, ARLINGTON HEIGHTS, IL, US, vol. 97, no. 3, 1 September 2006 (2006-09-01), pages 329-335, XP026959593, ISSN: 1081-1206 [retrieved on 2006-09-01]
- CAN ET AL.: "Effects of MK -886, a leukotriene biosynthesis inhibitor, in a rabbit model of endotoxic shock", EUROPEAN JOURNAL OF PHARMACOLOGY, vol. 350, 1998, pages 223-228, XP55170915,

## Description

The present invention relates to zafirlukast and its metabolites for use in the treatment of bacterial infections and as anti-bacterial and antibiotic agents.

Antibiotic resistance is a major problem in the global fight against infectious diseases. The continual adaption of bacterial species has resulted in an increase in antibiotic-resistant strains, including strains of bacteria that are considered to be multi-drug resistant (MDR).

The emergence of antibiotic resistant strains has been worsened by such behaviours as inappropriate prescribing of antibiotics, poor patient compliance (the patient stops taking the antibiotic when the symptoms subside and do not finish the course prescribed by their doctor) or the lack of control in the supply of antibiotics to the public, for example, in those countries where antibiotics are available over the counter without prescription. Antibiotic use in veterinary medicine may also play a role.

Common strains of antibiotic-resistant bacteria include methicillin-resistant *Staphylococcus aureus* (MRSA), *Streptococcus pneumoniae,* (resistant to penicillin and other beta-lactams) and multi-drug resistant *Enterococcus faecalis* and *Clostridium difficile,* to name but a few.

Leukotrienes are biologically active compounds derived from arachidonic acid. They were originally discovered in leukocytes, although they are also found in other immune cells. They are a family of eicosanoid inflammatory mediators. They are known to contribute to the pathophysiology of asthma and may cause or potentiate symptoms of increased mucus secretion, bronchoconstriction, air flow obstruction and infiltration of inflammatory cells into the airway wall. Leukotriene antagonists inhibit leukotriene receptors and are known for use in the treatment of asthma or bronchitis, although are less effective than corticosteroids.

Zafirlukast is a leukotriene antagonist used for the treatment of asthma, often in conjunction with an inhaled steroid and/or long-acting bronchodilator. It is marketed by AstraZeneca under the names Accolate, Accoleate and Vanticon. It is administered orally and has a plasma half-life of approximately 10 hours. Zafirlukast blocks the action of cysteinyl leukotrienes on CysLT1 receptors.

Pinault *et al* noted that zafirlukast inhibits the complexation of Lsr2 (a regulatory protein involved in multiple cellular processes, including cell wall biosynthesis) with DNA and growth of *Mycobacterium tuberculosis* (Pinault et al, Antimicrobial Agents and Chemotherapy, 2013, 57(5):2134-2140). In particular, the authors noted that zafirlukast inhibits the growth of *Mycobacterium smegmatis* and *Mycobacterium tuberculosis,* although it had no effect on the growth of E. *coli.* The authors also found that zafirlukast exhibits bactericidal activity against M. *smegmatis* but these effects were attributed to the inhibitory effect of zafirlukast on Lsr2.

WO 2013/055674 discloses methods of treating bacterial infections using zafirlukast, in particular *Mycobacterium* infections and *Corynebacterium* infections.

WO 2014/068320 discloses a ketone body inhibitor for use in the treatment or prevention of a disease or condition associated with gastrointestinal tract barrier impairment.

WO 2009/152454 discloses methods of treating organ specific infections in a host organism by administering compounds that target host receptors and/or host cellular signaling molecules to prevent a pathogen from infecting the organ.

Goldberg et al., 1986. Pediatric Research, vol. 20, no. 10, pages 1004-1008, studied the effect of a leukotriene antagonist, FPL 57231, on the early hemodynamic changes occurring secondary to an infusion of live group B streptococcus.

Schreiber et al., 1992, Pediatric Research, vol. 31, no. 2, pages 121-126, studied the pulmonary and systemic hemodynamic response of newborn lambs to injections of heat-killed type lb group B streptococcus.

Khoury et al:, 2006 Annals Of Allergy, Asthma & Immunology, vol. 97, no. 3, pages 329-335 discloses the effect of montelukast on bacterial sinusitis in allergic mice.

Can et al., 1998, European Journal Of Pharmacology, vol. 350, 1998, pages 223-228, discloses the effects of MK -886, a leukotriene biosynthesis inhibitor, in a rabbit model of endotoxic shock.

There is a need in the art for the provision of new antibiotics or the discovery of novel uses of known compounds as antibiotics to address the growing and serious problem of bacterial resistance to current treatment regimens. The ability to treat infections of antibiotic-resistant bacteria would be particularly advantageous. Lower dosages are also desirable.

The present inventors have surprisingly found that leukotriene antagonists, such as zafirlukast and derivatives thereof, have antimicrobial activity against a range of bacteria including a number of opportunistic Gram-positive organisms, such as *Staphylococcus aureus,* methicillin-resistant *Staphylococcus aureus, Enterococcus faecalis, Bacillus subtilis, Streptococcus pneumoniae,* and *Clostridium difficile.*

The present invention provides zafirlukast, a metabolite, or a pharmaceutically acceptable salt or ester thereof, for use as a bactericidal agent in the treatment of a Gram-positive bacterial infection, wherein:
the bacterial infection is an infection selected from the group consisting of a Staphylococcus spp., an Enterococcus spp., a Bacillus spp., a Streptococcus spp. and a Clostridium spp. infection; and
said metabolite of zafirlukast is selected from the group consisting of: N-[4-(5-Amino-1-methyl-1H-indol-3-ylmethyl)-3-methoxybenzoyl]-2-methyl-benzenesulfonamide, N-[3-[2-Methoxy-4-(toluene-2-sulfonylaminocarbonyl)benzyl]-1H-indol-5-yl]acetamide, N-[1-Hydroxymethyl-3-[2-methoxy-4-(toluene-2-sulfonylaminocarbonyl)-benzyl]-1H-indol-5-yl]acetamide, N-[3-[2-Methoxy-4-(toluene-2-sulfonylaminocarbonyl)benzyl]-1-methyl-1H-indol-5-yl]acetamide, [3-[2-Methoxy-4-(toluene-2-sulfonylaminocarbonyl)benzyl]-1H-indol-5-yl]carbamic acid cyclopentyl ester, [3-[2-Methoxy-4-(toluene-2-sulfonylaminocarbonyl)benzyl]-1-methyl-1H-indol-5-yl]carbamic acid hydroxycyclopentyl ester, [1-Hydroxymethyl-3-[2-methoxy-4-(toluene-2-sulfonylaminocarbonyl)-benzyl]-1H-indol-5-yl]carbamic acid cyclopentyl ester and [3-[2-Methoxy-4-(toluene-2-sulfonylaminocarbonyl)benzyl]-1H-indol-5-yl]carbamic acid hydroxycyclopentyl ester.

The present invention also provides a pharmaceutical composition comprising zafirlukast, a metabolite, or a pharmaceutically acceptable salt or ester thereof, and one or more pharmaceutically acceptable excipients, for use as a bactericidal agent in the treatment of a Gram-positive bacterial infection, wherein:
the bacterial infection is an infection selected from the group consisting of a Staphylococcus spp., an Enterococcus spp., a Bacillus spp., a Streptococcus spp. and a Clostridium spp. infection; and
wherein said metabolite of zafirlukast is selected from the group consisting of: N-[4-(5-Amino-1-methyl-1H-indol-3-ylmethyl)-3-methoxybenzoyl]-2-methyl-benzenesulfonamide, N-[3-[2-Methoxy-4-(toluene-2-sulfonylaminocarbonyl)benzyl]-1H-indol-5-yl]acetamide, N-[1-Hydroxymethyl-3-[2-methoxy-4-(toluene-2-sulfonylaminocarbonyl)-benzyl]-1H-indol-5-yl]acetamide, N-[3-[2-Methoxy-4-(toluene-2-sulfonylaminocarbonyl)benzyl]-1-methyl-1H-indol-5-yl]acetamide, [3-[2-Methoxy-4-(toluene-2-sulfonylaminocarbonyl)benzyl]-1H-indol-5-yl]carbamic acid cyclopentyl ester, [3-[2-Methoxy-4-(toluene-2-sulfonylaminocarbonyl)benzyl]-1-methyl-1H-indol-5-yl]carbamic acid hydroxycyclopentyl ester, [1-Hydroxymethyl-3-[2-methoxy-4-(toluene-2-sulfonylaminocarbonyl)-benzyl]-1H-indol-5-yl]carbamic acid cyclopentyl ester and [3-[2-Methoxy-4-(toluene-2-sulfonylaminocarbonyl)benzyl]-1H-indol-5-yl]carbamic acid hydroxycyclopentyl ester.

The compounds used in the present invention are particularly useful in the treatment of infections by Firmicutes, in particular Gram-positive Firmicutes. In some embodiments, the compounds are used to treat *Staphylococcus spp., Enterococcus spp., Bacillus spp., Streptococcus spp.* and *Clostridium spp.* infections. In some embodiments, the bacterial infections treated are infections by opportunistic bacteria, in particular, opportunistic Gram-positive bacteria. Nosocomial infections, including nosocomial Gram-positive infections, may be targeted in the present invention. The bacteria may be antibiotic-resistant or even multidrug-resistant strains. Specific infections that can be treated include *Staphylococcus aureus,* methicillin resistant *Staphylococcus aureus, Enterococcus faecalis, Bacillus subtilis, Streptococcus pneumoniae* and *Clostridium difficile* infections.

The bacterial infections may be chronic or acute bacterial infections, although the invention is particularly useful for the treatment of acute bacterial infections.

Zafirlukast has the following structure:

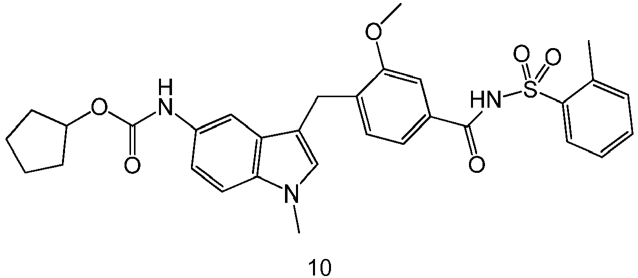

The terms "leukotriene antagonists" and "leukotriene receptor antagonists" are used interchangeably. They refer to compounds that block the action of cysteinyl leukotrienes at the CysLT1 receptor on target cells such as bronchial smooth muscle. Such compounds are also known as "leukasts".

In some embodiments of the invention, the compound used in the invention is zafirlukast or one of its metabolites. The systematic name for zafirlukast is [3-[2-Methoxy-4-(toluene-2-sulfonylaminocarbonyl)benzyl]-l-methyl-lH- indol-5-yl]carbamic acid cyclopentyl ester. Metabolites of zafirlukast for use in the invention include N-[4-(5-Amino-1-methyl-1 H-indol-3-ylmethyl)-3-methoxybenzoyl]-2-methyl-benzenesulfonamide, N-[3-[2-Methoxy-4-(toluene-2-sulfonylaminocarbonyl)benzyl]-1H-indol-5-yl]acetamide, N-[1-Hydroxymethyl-3-[2-methoxy-4-(toluene-2-sulfonylaminocarbonyl)-benzyl]-1H-indol-5-yl]acetamide, N-[3-[2-Methoxy-4-(toluene-2-sulfonylaminocarbonyl)benzyl]-1-methyl-1H-indol-5-yl]acetamide, [3-[2-Methoxy-4-(toluene-2-sulfonylaminocarbonyl)benzyl]-lH-indol-5-yl]carbamic acid cyclopentyl ester, [3-[2-Methoxy-4-(toluene-2-sulfonylaminocarbonyl)benzyl]-1-methyl-lH-indol-5-yl]carbamic acid hydroxycyclopentyl ester, [1-Hydroxymethyl-3-[2-methoxy-4-(toluene-2-sulfonylaminocarbonyl)-benzyl]-1H-indol-5-yl]carbamic acid cyclopentyl ester and [3-[2-Methoxy-4-(toluene-2-sulfonylaminocarbonyl)benzyl]-1H-indol-5-yl]carbamic acid hydroxycyclopentyl ester.

The compounds used in the invention may be in the form of pharmaceutically acceptable salts. Additionally, the compounds used in the invention may be formulated as pharmaceutical compositions comprising a compound used in the invention (or pharmaceutically acceptable salts, or esters thereof) and one or more pharmaceutically acceptable excipients.

In some embodiments of the invention, the infection treated using zafirlukast, a metabolite, or a pharmaceutically acceptable salt or ester thereof may be a skin, respiratory tract, alimentary canal or systemic infection. They may be infections in mammals, for example humans.

The compounds used in the present invention can be obtained by any suitable means known to a person of skill in the art. Some compounds, such as zafirlukast, are available for purchase from, for example, Sigma Aldrich (UK).

In embodiments of the invention, the zafirlukast, metabolite, or pharmaceutically acceptable salt or ester thereof, may be administered in combination with one or more other pharmaceutically active agents. The zafirlukast, metabolite, or pharmaceutically acceptable salt or ester thereof may be for simultaneous, separate or sequential administration with another pharmaceutically active agent. Generally, the additional pharmaceutically active agents will be present in a separate preparation, although they may be present in the same pharmaceutical composition.

The compounds or leukotriene antagonists used in the invention may be administered in combination with one or more anti-inflammatory agents. Such anti-inflammatory agents include glucocorticoids, such as prednisone, prednisolone, dexamethasone, methylprednisolone, budesonide, hydrocortisone, betamethasone, triamcinolone or fludrocortisone. A preferred glucocorticoid may be prednisolone. Generally, the compounds used in the present invention will not be administered with NSAIDs.

The compounds or leukotriene antagonists used in the invention may be for administration in combination with asthma therapy. Such asthma therapies include glucocorticoids, β-adrenergic agonists (short-acting, such as salbutamol, or long-acting, such as salmeterol or formoterol) or anti-cholinergic medications. In some embodiments, the zafirlukast is not administered with any such asthma therapies. Indeed, in some embodiments, the leukotriene antagonist, or compound of any one of Formulae I to III, is the only pharmaceutically active agent administered to the patient to treat the bacterial infection.

The compounds or leukotriene antagonists used in the invention may be administered in combination with one or more antibacterial agents. Such antibacterial agents include aminoglycosides, cephalosporins, macrolides, penicillins, quinolones, sulfonamides or tetracyclines. Aminoglycosides include amikacin, gentamycin, kanamycin, neomycin and tobramycin. Macrolides include azithromycin, clarithromycin, erythromycin or telithromycin. Penicillins include amoxicillin, ampicillin, flucloxacillin, methicillin, nafcillin, oxacillin, penicillin G, penicillin V or piperacillin. Quinolones include ciprofloxacin or levofloxacin. Tetracyclines include doxycycline and tetracycline. The additional antibiotics may be effective against Gram-positive bacteria. The additional antibiotics may be an antibiotic to which the bacterial infection is resistant, for example β-lactam antibiotics such as methicillin.

In other embodiments, the zafirlukast, metabolite, or pharmaceutically acceptable salt or ester thereof, is the only pharmaceutically active agent administered to the patient to treat the bacterial infection. In some embodiments, the compound used in the invention is the only antibiotic or antibacterial agent administered to the patient, although other pharmaceutically active agents may be administered.

In a second aspect of the invention there is provided a pharmaceutical composition comprising zafirlukast, metabolite, or pharmaceutically acceptable salt or ester thereof and one or more pharmaceutically acceptable excipients for use in the treatment of bacterial infections. There is also provided a pharmaceutical composition comprising zafirlukast, metabolite, or pharmaceutically acceptable salt or ester thereof and one or more pharmaceutically acceptable excipients, for use in the treatment of bacterial infections.

The pharmaceutical composition may be adapted for administration by any appropriate route, for example by the oral (including buccal or sublingual), rectal, nasal, topical (including buccal, sublingual or transdermal), vaginal or parenteral (including subcutaneous, intramuscular, intravenous or intradermal) route. Such compositions may be prepared by any method known in the art of pharmacy, for example by admixing the active ingredient with the carrier(s) or excipient(s) under sterile conditions.

Pharmaceutical compositions adapted for oral administration may be presented as discrete units such as capsules or tablets; as powders or granules; as solutions, syrups or suspensions (in aqueous or non-aqueous liquids; or as edible foams or whips; or as emulsions).

Suitable excipients for tablets or hard gelatine capsules include lactose, maize starch or derivatives thereof, stearic acid or salts thereof. Suitable excipients for use with soft gelatine capsules include for example vegetable oils, waxes, fats, semi-solid, or liquid polyols etc. The excipient can be lactose or microcrystalline cellulose.

For the preparation of solutions and syrups, excipients which may be used include, for example water, polyols and sugars. For the preparation of suspensions oils (e.g. vegetable oils) may be used to provide oil-in-water or water in oil suspensions.

The leukotriene antagonists, such as those of any of Formulae I to III, are particularly suited to oral and systemic administration (such as administration by injection, including intravenous and/or intra-arterial administration). Administration by inhalation is also a preferred route (for example when formulated with lactose).

Pharmaceutical compositions adapted for transdermal administration may be presented as discrete patches intended to remain in intimate contact with the epidermis of the recipient for a prolonged period of time. For example, the active ingredient may be delivered from the patch by iontophoresis as generally described in Pharmaceutical Research, 3 (6), page 318 (1986).

Pharmaceutical compositions adapted for topical administration may be formulated as ointments, creams, suspensions, lotions, powders, solutions, pastes, gels, sprays, aerosols or oils. For infections of the eye or other external tissues, for example mouth and skin, the compositions are suitably applied as a topical ointment or cream. When formulated in an ointment, the active ingredient may be employed with either a paraffinic or a water-miscible ointment base. Alternatively, the active ingredient may be formulated in a cream with an oil-in-water cream base or a water-in-oil base. Pharmaceutical compositions adapted for topical administration to the eye include eye drops wherein the active ingredient is dissolved or suspended in a suitable carrier, especially an aqueous solvent. Pharmaceutical compositions adapted for topical administration in the mouth include lozenges, pastilles and mouth washes.

Pharmaceutical compositions adapted for rectal administration may be presented as suppositories or enemas. Pharmaceutical compositions adapted for nasal administration wherein the carrier is a solid include a coarse powder having a particle size for example in the range 20 to 500 microns. Suitable compositions wherein the carrier is a liquid, for administration as a nasal spray or as nasal drops, include aqueous or oil solutions of the active ingredient. Pharmaceutical compositions adapted for vaginal administration may be presented as pessaries, tampons, creams, gels, pastes, foams or spray formulations. Pharmaceutical compositions adapted for administration by inhalation include powders and fine particle dusts or mists which may be generated by means of various types of metered dose pressurised aerosols, nebulizers or insufflators.

Pharmaceutical compositions adapted for parenteral administration include aqueous and non-aqueous sterile injection solution which may contain anti-oxidants, buffers, bacteriostats and solutes which render the formulation substantially isotonic with the blood of the intended recipient; and aqueous and non-aqueous sterile suspensions which may include suspending agents and thickening agents. Excipients which may be used for injectable solutions include water, alcohols, polyols, glycerine and vegetable oils, for example. The compositions may be presented in unit-dose or multi-dose containers, for example sealed ampoules and vials, and may be stored in a freeze-dried (lyophilized) condition requiring only the addition of the sterile liquid carried, for example water for injections, immediately prior to use. Extemporaneous injection solutions and suspensions may be prepared from sterile powders, granules and tablets.

The pharmaceutical compositions may contain preserving agents, solubilising agents, stabilising agents, wetting agents, emulsifiers, sweeteners, colourants, odourants, salts (substances of the present invention may themselves be provided in the form of a pharmaceutically acceptable salt), buffers, coating agents or antioxidants. They may also contain therapeutically active agents in addition to the substance of the present invention. References herein to leukotriene antagonists include pharmaceutically acceptable salts or esters thereof. Similarly, references to compounds of any of Formulae I to III include pharmaceutically acceptable salts or esters thereof. Such salts include hydrochloride (HCI), mesylate, maleate, chloride, bromide, citrate, tartrate, sulphate, and phosphate, including any suitable cation (sodium, calcium, benzathine, magnesium, ammonium, zinc, potassium and so on). Compounds used in the invention may include a carboxylic acid group; for such compounds, a salt can be formed by decomposition of the carboxylic acid to form a carboxylate.

In embodiments in which the compounds used in the invention are formulated as compositions for oral administration, the compositions may further comprise croscarmellose sodium, lactose, magnesium stearate, microcrystalline cellulose, povidone, hypromellose, and titanium dioxide. The compositions may be tablets, such as film-coated tablets. In embodiments of the invention in which the compounds used are formulated as compositions for oral administration, they may be formulated as sustained- or controlled-release formulations.

Dosages of the pharmaceutical compositions of the present invention can vary between wide limits, depending upon the disease or disorder to be treated, the age and condition of the individual to be treated, etc. and a physician will ultimately determine appropriate dosages to be used. Such compositions may be formulated for human or for veterinary medicine. The present application should be interpreted as applying equally to humans as well as to animals, unless the context clearly implies otherwise.

In embodiments in which the compounds used in the invention are formulated as compositions for oral administration, the compositions may comprise between 5 and 50 mg of the leukotriene receptor antagonists (or a compound of any one of Formulae I to III), for example between 10 and 20mg. In some embodiments, the amount to be administered may be between 0.05 and 100 mg/kg, for example between 0.05 and 50 mg/kg, 0.05 and 25 mg/kg, 0.05 and 5 mg/kg, 0.05 and 1 mg/kg or 0.05 and 0.5 mg/kg.

The pharmaceutical compositions may further comprise additional pharmaceutically active agents, for example antibacterial agents, anti-inflammatory agents or agents used in asthma therapy. In other embodiments, the leukotriene antagonists, or compound of any one of Formulae I to III, is the only pharmaceutically active agents present in the pharmaceutical composition.

The compound or pharmaceutical composition may be administered by any suitable route, for example the oral route or systemic route (such as by injection, for example intravenous injection), or by inhalation. Additional pharmaceutically active agents may also be administered to treat the bacterial infection. In other embodiments, the leukotriene antagonist or a compound of any one of Formulae I to III may be the only pharmaceutically active agent administered to the patient to treat the bacterial infection.

Methods of treatment may include a step of selecting a patient for treatment. For example, the method may include a step of determining whether a patient will be susceptible to treatment. Such steps may include first determining the presence (or absence) of a bacterial infection and administering the compound used in the disclosure only if a bacterial infection is detected. In particular, treatment might only be initiated if the patient is infected with a certain type of bacterial infection, for example infection by Gram-positive bacteria, a Firmicute or a Gram-positive Firmicute. Nosocomial infections, including nosocomial Gram-positive nosocomial infections, may be targeted in the present invention.

The decision whether or not to treat may be based on the present of an antibiotic-resistant bacterial infection, or even multidrug-resistant strains. Specific infections that can be treated include *Staphylococcus aureus,* methicillin resistant *Staphylococcus aureus, Enterococcus faecalis, Bacillus subtilis, Streptococcus pneumoniae* and *Clostridium difficile* infections. In some embodiments, treatment may be initiated if an infection with bacterial species that do not encode Lsr2, or an Lsr2-homologue, is detected. In some embodiments, treatment is not initiated if a *Mycobacterium spp.* or *Corynebacterium spp.* infection is detected or suspected. In other embodiments, treatment is not initiated if an actinobacterial infection is detected or suspected, for example a *Streptomyces spp., Nocardia spp.* or *Rhodococcus spp.* infection.

The step of determining the presence of a bacterial infection may include testing for the infection in question. It may include testing for the presence of an infection by Gram-positive bacteria, for example an infection by *Staphylococcus aureus,* methicillin resistant *Staphylococcus aureus, Enterococcus faecalis, Bacillus subtilis, Streptococcus pneumoniae* or *Clostridium difficile,* and administering a compound used in the invention accordingly if such an infection is detected or suspected. In other embodiments, the step of determining the presence of a bacterial infection may include testing for the presence of a *Mycobacterium spp.* and/or *Corynebacterium spp.* infection, with the decision to treat the patient by administering a compound used the invention only being taken in the absence of such infection. In other embodiments, the step of determining the presence of a bacterial infection may include testing for the presence of an actinobacterial, for example a *Streptomyces spp., Nocardia spp.* or *Rhodococcus spp.* infection, with the decision to treat the patient by administering a compound used the invention only being taken in the absence of such infection.

In a fourth aspect of the disclosure there is provided the use of a leukotriene antagonist, or the use of a compound of any one of Formulae I to III, or the use of a pharmaceutical composition comprising a leukotriene antagonist or a compound of any one of Formulae I to III, in the manufacture of a medicament for the treatment of bacterial infection.

In a further aspect of the invention there is provided zafirlukast, metabolite, or pharmaceutically acceptable salt or ester thereof, or a pharmaceutical composition comprising zafirlukast, metabolite, or pharmaceutically acceptable salt or ester thereof, for use in the treatment of a bacterial infection accompanying inflammatory lung disease or (chronic) asthma, or obstructive lung diseases. The compound used in the invention is administered as an antibiotic, in particular a bactericidal antibiotic. The inflammatory lung disease may be asthma, cystic fibrosis, emphysema, chronic obstructive pulmonary disorder (COPD) or acute respiratory distress syndrome (ARDS). In some embodiments, the respiratory tract infection is an upper respiratory tract infection. In other embodiments, the respiratory tract infection is a lower respiratory tract infection. In some embodiments, the disease is not tuberculosis. Generally, the bacterial infection accompanying inflammatory lung disease or chronic asthma, or obstructive lung diseases is not a *Mycobacterium* or *Corynebacterium* infection.

The compounds or pharmaceutical compositions may be used in methods of preventing, reducing, inhibiting and/or controlling bacterial infections, as well as treating such infections. They may be for prophylactic administration.

In one embodiment of the invention there is provided zafirlukast for use in in the treatment of a bacterial infection, wherein the bacteria is selected from the group consisting of *Staphylococcus aureus,* methicillin resistant *Staphylococcus aureus, Enterococcus faecalis, Bacillus subtilis, Streptococcus pneumoniae,* and *Clostridium difficile.* The zafirlukast is for oral administration to a patient in need thereof.

Preferred features for the second and subsequent aspects of the invention are as provided for the first aspect, *mutatis mutandis.*

The present invention will now be described by way of reference to the following Examples which are present for the purposes of reference. In the examples, reference is made to a number of drawings in which:
Figure 1 shows the effect of decreasing amounts of zafirlukast on *Bacillus subtilis.* In the figure, 1 = DMSO alone (control) and 2 to 4 = decreasing amounts of zafirlukast (10, 5 and 2 µg, respectively).
Figure 2 shows the bactericidal activity of zafirlukast on S. *aureus* and B. *subtilis.*

### Example 1

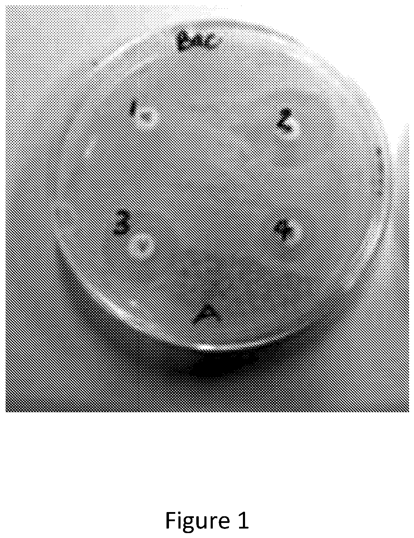

Zafirlukast (Sigma Aldrich, UK) was resuspended in Dimethyl Sulfoxide to a concentration of 16mM. A minimum inhibitory concentration (MIC) was conducted using various bacterial broth solutions containing between the 0 and 4 mM zafirlukast. 10⁵ cfu/mL of test organism was added to each tube before incubation overnight at 37°C. The MIC was taken to be the concentration at which no visible growth was observed. In addition, zone inhibition studies were carried out using standard techniques. The results are shown in Figure 1.

The bacterial species tested included *Staphylococcus aureus,* methicillin resistant *Staphylococcus aureus, Enterococcus faecalis, Bacillus subtilis, Streptococcus pneumoniae,* and *Clostridium difficile.*

The MICs were as follows:

### Staphylococcus aureus:

Zafirlukast = 0.03mM
Ciprofloxacin = 0.003mM
Ceftriaxone = 0.002mM

### Bacillus subtilis

Zafirlukast = 0.006mM
Ceftriaxone = >0.029mM
Ciprofloxacin = >0.012mM,

### MRSA

Zafirlukast = 0.006mM
Ciprofloxacin = average 0.04mM
Ceftriaxone=0.022mM

MIC is well known to the skilled person and is defined as the lowest concentration of agent which prevents visible growth of the bacterial strain under investigation.

### Example 2

Bactericidal activity of zafirlukast.

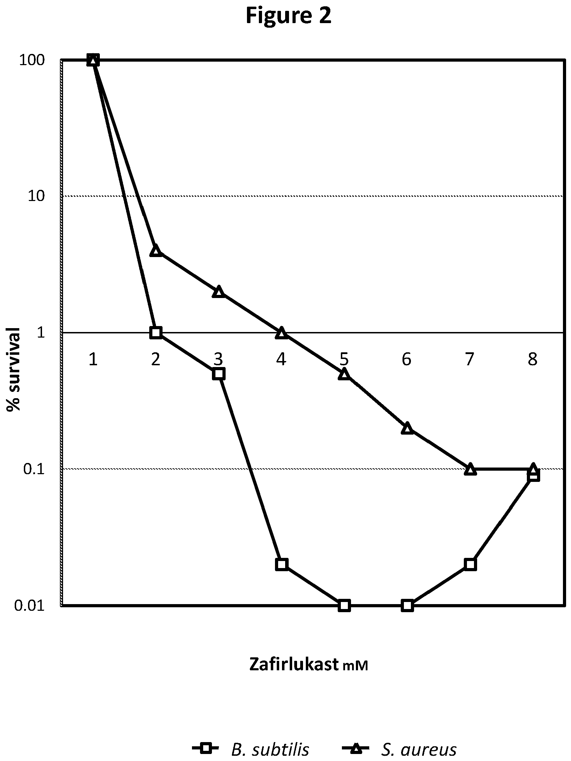

105 cfu/mL of bacterial culture was incubated in the presence of various concentrations of zafirlukast for a period of 3hrs at 37°C. At the end of this incubation, viable counts were taken and results recoded as percentage survival (as compared to the drug free control). The results are shown in Figure 2.

## Claims

1. Zafirlukast, a metabolite, or a pharmaceutically acceptable salt or ester thereof, for use as a bactericidal agent in the treatment of a Gram-positive bacterial infection, wherein:
the bacterial infection is an infection selected from the group consisting of a *Staphylococcus spp.,* an *Enterococcus spp.,* a *Bacillus spp.,* a *Streptococcus spp.* and a *Clostridium spp.* infection; and
said metabolite of zafirlukast is selected from the group consisting of: N-[4-(5-Amino-1-methyl-1H-indol-3-ylmethyl)-3-methoxybenzoyl]-2-methyl-benzenesulfonamide, N-[3-[2-Methoxy-4-(toluene-2-sulfonylaminocarbonyl)benzyl]-1H-indol-5-yl]acetamide, N-[1-Hydroxymethyl-3-[2-methoxy-4-(toluene-2-sulfonylaminocarbonyl)-benzyl]-1H-indol-5-yl]acetamide, N-[3-[2-Methoxy-4-(toluene-2-sulfonylaminocarbonyl)benzyl]-1-methyl-1H-indol-5-yl]acetamide, [3-[2-Methoxy-4-(toluene-2-sulfonylaminocarbonyl)benzyl]-IH-indol-5-yl]carbamic acid cyclopentyl ester, [3-[2-Methoxy-4-(toluene-2-sulfonylaminocarbonyl)benzyl]-1-methyl-lH-indol-5-yl]carbamic acid hydroxycyclopentyl ester, [1-Hydroxymethyl-3-[2-methoxy-4-(toluene-2-sulfonylaminocarbonyl)-benzyl]-1H-indol-5-yl]carbamic acid cyclopentyl ester and [3-[2-Methoxy-4-(toluene-2-sulfonylaminocarbonyl)benzyl]-1H-indol-5-yl]carbamic acid hydroxycyclopentyl ester.

2. A pharmaceutical composition comprising zafirlukast, a metabolite, or a pharmaceutically acceptable salt or ester thereof, and one or more pharmaceutically acceptable excipients, for use as a bactericidal agent in the treatment of a Gram-positive bacterial infection, wherein:
the bacterial infection is an infection selected from the group consisting of a *Staphylococcus spp.,* an *Enterococcus spp.,* a *Bacillus spp.,* a *Streptococcus spp.* and a *Clostridium spp.* infection; and
wherein said metabolite of zafirlukast is selected from the group consisting of: N-[4-(5-Amino-1-methyl-1H-indol-3-ylmethyl)-3-methoxybenzoyl]-2-methyl-benzenesulfonamide, N-[3-[2-Methoxy-4-(toluene-2-sulfonylaminocarbonyl)benzyl]-1H-indol-5-yl]acetamide, N-[1-Hydroxymethyl-3-[2-methoxy-4-(toluene-2-sulfonylaminocarbonyl)-benzyl]-1H-indol-5-yl]acetamide, N-[3-[2-Methoxy-4-(toluene-2-sulfonylaminocarbonyl)benzyl]-1-methyl-1H-indol-5-yl]acetamide, [3-[2-Methoxy-4-(toluene-2-sulfonylaminocarbonyl)benzyl]-IH-indol-5-yl]carbamic acid cyclopentyl ester, [3-[2-Methoxy-4-(toluene-2-sulfonylaminocarbonyl)benzyl]-1-methyl-IH-indol-5-yl]carbamic acid hydroxycyclopentyl ester, [1-Hydroxymethyl-3-[2-methoxy-4-(toluene-2-sulfonylaminocarbonyl)-benzyl]-1H-indol-5-yl]carbamic acid cyclopentyl ester and [3-[2-Methoxy-4-(toluene-2-sulfonylaminocarbonyl)benzyl]-1H-indol-5-yl]carbamic acid hydroxycyclopentyl ester.

3. Zafirlukast, a metabolite, or a pharmaceutically acceptable salt or ester thereof for use according to claim 1, or the pharmaceutical composition for use according to claim 2, wherein the bacterial infection is an opportunistic or nosocomial bacterial infection.

4. Zafirlukast, a metabolite, or a pharmaceutically acceptable salt or ester thereof for use according to any of claims 1 or 3, or the pharmaceutical composition for use according to claim 2 or 3, wherein the infection is selected from the group consisting of a *Staphylococcus aureus,* methicillin resistant *Staphylococcus aureus, Enterococcus faecalis, Bacillus subtilis, Streptococcus pneumoniae,* and *Clostridium difficile* infection.

5. Zafirlukast, a metabolite, or a pharmaceutically acceptable salt or ester thereof for use according to any of claims 1, 3 or 4, or the pharmaceutical composition for use according to any of claims 2 to 4, wherein the zafirlukast, metabolite, or a pharmaceutically acceptable salt or ester thereof or pharmaceutical composition is for oral or systemic administration, or for administration by inhalation.

6. The pharmaceutical composition for use according to any one of claims 2 to 5, wherein the pharmaceutical composition comprises one or more other pharmaceutically active agents.

7. The pharmaceutical composition for use according to claim 6, wherein the one or more pharmaceutically active agents is an antibacterial agent, an anti-inflammatory agent, or an agent used in asthma therapy.

8. Zafirlukast, a metabolite, or a pharmaceutically acceptable salt or ester thereof for use according to claims 1, 3, 4 or 5, or the pharmaceutical composition for use according to any of claims 2 to 5, wherein the zafirlukast, metabolite, or a pharmaceutically acceptable salt or ester thereof or pharmaceutical composition is for simultaneous, separate or sequential administration with another pharmaceutically active agent.

9. Zafirlukast, a metabolite, or a pharmaceutically acceptable salt or ester thereof for use according to claim 8 or the pharmaceutical composition for use according to claim 8, wherein the pharmaceutically active agent is an antibacterial agent, an anti-inflammatory agent, or an agent used in asthma therapy.

## Patentansprüche

1. Zafirlukast, ein Metabolit oder ein pharmazeutisch unbedenkliches Salz oder ein pharmazeutisch unbedenklicher Ester davon, zur Verwendung als bakterizides Mittel bei der Behandlung einer Infektion mit grampositiven Bakterien, wobei:
es sich bei der Bakterieninfektion um eine Infektion aus der Gruppe bestehend aus einer Infektion mit *Staphylococcus spp., Enterococcus spp., Bacillus spp., Streptococcus spp.* und *Clostridium spp.* handelt und
der Metabolit von Zafirlukast aus der Gruppe bestehend aus N-[4-(5-Amino-1-methyl-1H-indol-3-ylmethyl)-3-methoxybenzoyl]-2-methylbenzolsulfon-amid, N-[3-[2-Methoxy-4-(toluol-2-sulfonylamino-carbonyl)benzyl]-1H-indol-5-yl]acetamid, N-[1-Hydroxymethyl-3-[2-methoxy-4-(toluol-2-sulfonyl-aminocarbonyl)benzyl]-1H-indol-5-yl]acetamid, N-[3-[2-Methoxy-4-(toluol-2-sulfonylaminocarbonyl)-benzyl]-1-methyl-1H-indol-5-yl]acetamid, [3-[2-Methoxy-4-(toluol-2-sulfonylaminocarbonyl)benzyl]-1H-indol-5-yl]carbamidsäurecyclopentylester, [3-[2-Methoxy-4-(toluol-2-sulfonylaminocarbonyl)-benzyl]-1-methyl-1H-indol-5-yl]carbamidsäure-hydroxycyclopentylester, [1-Hydroxymethyl-3-[2-methoxy-4-(toluol-2-sulfonylaminocarbonyl)benzyl]-1H-indol-5-yl]carbamidsäurecyclopentylester und [3-[2-Methoxy-4-(toluol-2-sulfonylaminocarbonyl)-benzyl]-1H-indol-5-yl]carbamidsäurehydroxycyclo-pentylester ausgewählt ist.

2. Pharmazeutische Zusammensetzung, umfassend Zafirlukast, einen Metaboliten oder ein pharmazeutisch unbedenkliches Salz oder einen pharmazeutisch unbedenklichen Ester davon und einen oder mehrere pharmazeutisch unbedenkliche Hilfsstoffe, zur Verwendung als bakterizides Mittel bei der Behandlung einer Infektion mit grampositiven Bakterien, wobei:
es sich bei der Bakterieninfektion um eine Infektion aus der Gruppe bestehend aus einer Infektion mit *Staphylococcus spp., Enterococcus spp., Bacillus spp., Streptococcus spp.* und *Clostridium spp.* handelt und
der Metabolit von Zafirlukast aus der Gruppe bestehend aus N-[4-(5-Amino-1-methyl-1H-indol-3-ylmethyl)-3-methoxybenzoyl]-2-methylbenzolsulfon-amid, N-[3-[2-Methoxy-4-(toluol-2-sulfonylamino-carbonyl)benzyl]-1H-indol-5-yl]acetamid, N-[1-Hydroxymethyl-3-[2-methoxy-4-(toluol-2-sulfonyl-aminocarbonyl)benzyl]-1H-indol-5-yl]acetamid, N-[3-[2-Methoxy-4-(toluol-2-sulfonylaminocarbonyl)-benzyl]-1-methyl-1H-indol-5-yl]acetamid, [3-[2-Methoxy-4-(toluol-2-sulfonylaminocarbonyl)benzyl]-1H-indol-5-yl]carbamidsäurecyclopentylester, [3-[2-Methoxy-4-(toluol-2-sulfonylaminocarbonyl)-benzyl]-1-methyl-1H-indol-5-yl]carbamidsäure-hydroxycyclopentylester, [1-Hydroxymethyl-3-[2-methoxy-4-(toluol-2-sulfonylaminocarbonyl)benzyl]-1H-indol-5-yl]carbamidsäurecyclopentylester und [3-[2-Methoxy-4-(toluol-2-sulfonylaminocarbonyl)-benzyl]-1H-indol-5-yl]carbamidsäurehydroxycyclo-pentylester ausgewählt ist.

3. Zafirlukast, ein Metabolit oder ein pharmazeutisch unbedenkliches Salz oder ein pharmazeutisch unbedenklicher Ester davon zur Verwendung nach Anspruch 1 oder pharmazeutische Zusammensetzung zur Verwendung nach Anspruch 2, wobei es sich bei der Bakterieninfektion um eine opportunistische oder nosokomiale Bakterieninfektion handelt.

4. Zafirlukast, ein Metabolit oder ein pharmazeutisch unbedenkliches Salz oder ein pharmazeutisch unbedenklicher Ester davon zur Verwendung nach einem der Ansprüche 1 oder 3 oder pharmazeutische Zusammensetzung zur Verwendung nach Anspruch 2 oder 3, wobei die Infektion aus der Gruppe bestehend aus einer Infektion mit *Staphylococcus aureus,* methicillinresistentem *Staphylococcus aureus, Enterococcus faecalis, Bacillus subtilis, Streptococcus pneumoniae* und *Clostridium difficile* ausgewählt ist.

5. Zafirlukast, ein Metabolit oder ein pharmazeutisch unbedenkliches Salz oder ein pharmazeutisch unbedenklicher Ester davon zur Verwendung nach einem der Ansprüche 1, 3 oder 4 oder pharmazeutische Zusammensetzung zur Verwendung nach einem der Ansprüche 2 bis 4, wobei das Zafirlukast, der Metabolit oder ein pharmazeutisch unbedenkliches Salz oder ein pharmazeutisch unbedenklicher Ester davon oder die pharmazeutische Zusammensetzung zur oralen oder systemischen Verabreichung oder zur Verabreichung durch Inhalation vorgesehen ist.

6. Pharmazeutische Zusammensetzung zur Verwendung nach einem der Ansprüche 2 bis 5, wobei die pharmazeutische Zusammensetzung einen oder mehrere andere pharmazeutische Wirkstoffe umfasst.

7. Pharmazeutische Zusammensetzung zur Verwendung nach Anspruch 6, wobei es sich bei dem pharmazeutischen Wirkstoff bzw. den pharmazeutischen Wirkstoffen um ein antibakterielles Mittel, ein entzündungshemmendes Mittel oder ein Mittel, das bei der Asthmatherapie verwendet wird, handelt.

8. Zafirlukast, ein Metabolit oder ein pharmazeutisch unbedenkliches Salz oder ein pharmazeutisch unbedenklicher Ester davon zur Verwendung nach den Ansprüchen 1, 3, 4 oder 5 oder pharmazeutische Zusammensetzung zur Verwendung nach einem der Ansprüche 2 bis 5, wobei das Zafirlukast, der Metabolit oder ein pharmazeutisch unbedenkliches Salz oder ein pharmazeutisch unbedenklicher Ester davon oder die pharmazeutische Zusammensetzung zur gleichzeitigen, separaten oder aufeinanderfolgenden Verabreichung mit einem anderen pharmazeutischen Wirkstoff vorgesehen ist.

9. Zafirlukast, ein Metabolit oder ein pharmazeutisch unbedenkliches Salz oder ein pharmazeutisch unbedenklicher Ester davon nach Anspruch 8 oder pharmazeutische Zusammensetzung zur Verwendung nach Anspruch 8, wobei es sich bei dem pharmazeutischen Wirkstoff um ein antibakterielles Mittel, ein entzündungshemmendes Mittel oder ein Mittel, das bei der Asthmatherapie verwendet wird, handelt.

## Revendications

1. Zafirlukast, métabolite, ou sel ou ester pharmaceutiquement acceptable correspondant, pour une utilisation en tant qu'agent bactéricide dans le traitement d'une infection par des bactéries Gram positives :
l'infection bactérienne étant une affection choisie dans le groupe constitué par une infection par *Staphylococcus spp.,* une infection par *Enterococcus spp.,* une infection par *Bacillus* spp., une infection par *Streptococcus spp.,* et une infection par *Clostridium spp* ; et
ledit métabolite de zafirlukast étant choisi dans le groupe constitué par : N-[4-(5-amino-1-méthyl-1H-indol-3-ylméthyl)-3-méthoxybenzoyl]-2-méthyl-benzènesulfonamide, N-[3-[2-méthoxy-4-(toluène-2-sulfonylaminocarbonyl)benzyl]-1H-indol-5-yl]acétamide, N-[1-hydroxyméthyl-3-[2-méthoxy-4-(toluène-2-sulfonylaminocarbonyl)-benzyl]-1H-indol-5-yl]acétamide, N-[3-[2-méthoxy-4-(toluène-2-sulfonylaminocarbonyl)benzyl]-1-méthyl-1H-indol-5-yl]acétamide, [3-[2-méthoxy-4-(toluène-2-sulfonylaminocarbonyl)benzyl]-1H-indol-5-yl]carbamate de cyclopentyle, [3-[2-méthoxy-4-(toluène-2-sulfonylaminocarbonyl)benzyl]-1-méthyl-1H-indol-5-yl]carbamate d'hydroxycyclopentyle, [1-hydroxyméthyl-3-[2-méthoxy-4-(toluène-2-sulfonylaminocarbonyl)-benzyl]-1H-indol-5-yl]carbamate de cyclopentyle et [3-[2-méthoxy-4-(toluène-2-sulfonylaminocarbonyl)benzyl]-1H-indol-5-yl]carbamate d'hydroxycyclopentyle.

2. Composition pharmaceutique comprenant du zafirlukast, un métabolite, ou un sel ou un ester pharmaceutiquement acceptable correspondant, et un ou plusieurs excipients pharmaceutiquement acceptables, pour une utilisation en tant qu'agent bactéricide dans le traitement d'une infection par des bactéries Gram positives :
l'infection bactérienne étant une affection choisie dans le groupe constitué par une infection par *Staphylococcus spp.,* une infection par *Enterococcus spp.,* une infection par *Bacillus spp.,* une infection par *Streptococcus spp.,* et une infection par *Clostridium spp* ; et
ledit métabolite de zafirlukast étant choisi dans le groupe constitué par : N-[4-(5-amino-1-méthyl-1H-indol-3-ylméthyl)-3-méthoxybenzoyl]-2-méthyl-benzènesulfonamide, N-[3-[2-méthoxy-4-(toluène-2-sulfonylaminocarbonyl)benzyl]-1H-indol-5-yl]acétamide, N-[1-hydroxyméthyl-3-[2-méthoxy-4-(toluène-2-sulfonylaminocarbonyl)-benzyl]-1H-indol-5-yl]acétamide, N-[3-[2-méthoxy-4-(toluène-2-sulfonylaminocarbonyl)benzyl]-1-méthyl-1H-indol-5-yl]acétamide, [3-[2-méthoxy-4-(toluène-2-sulfonylaminocarbonyl)benzyl]-1H-indol-5-yl]carbamate de cyclopentyle, [3-[2-méthoxy-4-(toluène-2-sulfonylaminocarbonyl)benzyl]-1-méthyl-1H-indol-5-yl]carbamate d'hydroxycyclopentyle, [1-hydroxyméthyl-3-[2-méthoxy-4-(toluène-2-sulfonylaminocarbonyl)-benzyl]-1H-indol-5-yl]carbamate de cyclopentyle et [3-[2-méthoxy-4-(toluène-2-sulfonylaminocarbonyl)benzyl]-1H-indol-5-yl]carbamate d'hydroxycyclopentyle.

3. Zafirlukast, métabolite, ou sel ou ester pharmaceutiquement acceptable correspondant pour une utilisation selon la revendication 1, ou composition pharmaceutique pour une utilisation selon la revendication 2, l'infection bactérienne étant une infection bactérienne opportuniste ou nosocomiale.

4. Zafirlukast, métabolite, ou sel ou ester pharmaceutiquement acceptable correspondant pour une utilisation selon l'une quelconque des revendications 1 et 3, ou composition pharmaceutique pour une utilisation selon la revendication 2 ou 3, l'infection étant choisie dans le groupe constitué par une infection par *Staphylococcus aureus,* une infection par *Staphylococcus aureus* résistant à la méthicilline, une infection par *Enterococcus faecalis,* une infection par *Bacillus subtilis,* une infection par *Streptococcus pneumoniae,* et une infection par *Clostridium difficile.*

5. Zafirlukast, métabolite, ou sel ou ester pharmaceutiquement acceptable correspondant pour une utilisation selon l'une quelconque des revendications 1, 3 et 4, ou composition pharmaceutique pour une utilisation selon l'une quelconque des revendications 2 à 4, le zafirlukast, un métabolite, ou un sel ou un ester pharmaceutiquement acceptable correspondant ou une composition pharmaceutique étant destiné(e) à une administration orale ou systémique, ou destiné(e) à une administration par inhalation.

6. Composition pharmaceutique pour une utilisation selon l'une quelconque des revendications 2 à 5, la composition pharmaceutique comprenant un ou plusieurs autres agents pharmaceutiquement actifs.

7. Composition pharmaceutique pour une utilisation selon la revendication 6, l'agent ou les agents pharmaceutiquement actifs étant un agent antibactérien, un agent anti-inflammatoire, ou un agent utilisé dans une thérapie de l'asthme.

8. Zafirlukast, métabolite, ou sel ou ester pharmaceutiquement acceptable correspondant pour une utilisation selon la revendication 1, 3, 4 ou 5, ou composition pharmaceutique pour une utilisation selon l'une quelconque des revendications 2 à 5, le zafirlukast, un métabolite, ou un sel ou un ester pharmaceutiquement acceptable correspondant ou une composition pharmaceutique étant destiné(e) à une administration simultanée, séparée ou séquentielle avec un autre agent pharmaceutiquement actif.

9. Zafirlukast, métabolite, ou sel ou ester pharmaceutiquement acceptable correspondant pour une utilisation selon la revendication 8 ou composition pharmaceutique pour une utilisation selon la revendication 8, l'agent pharmaceutiquement actif étant un agent antibactérien, un agent anti-inflammatoire, ou un agent utilisé dans une thérapie de l'asthme.


### SEQUENCE LISTING

<110> University of East London
<120> Leukotriene receptor antagonists and their derivatives for use as antibacterial agents
<130> P59029WO/TCL
<150> GB1321089.3
   <151> 2013-11-29
<160> 1
<170> PatentIn version 3.5
<210> 1
   <211> 112
   <212> PRT
   <213> Mycobacterium tuberculosis
<400> 1
   Met Ala Lys Lys Val Thr Val Thr Leu Val Asp Asp Phe Asp Gly Ser
   1               5                   10                  15
   Gly Ala Ala Asp Glu Thr Val Glu Phe Gly Leu Asp Gly Val Thr Tyr
               20                  25                  30
   Glu Ile Asp Leu Ser Thr Lys Asn Ala Thr Lys Leu Arg Gly Asp Leu
           35                  40                  45
   Lys Gln Trp Val Ala Ala Gly Arg Arg Val Gly Gly Arg Arg Arg Gly
       50                  55                  60
   Arg Ser Gly Ser Gly Arg Gly Arg Gly Ala Ile Asp Arg Glu Gln Ser
   65                  70                  75                  80
   Ala Ala Ile Arg Glu Trp Ala Arg Arg Asn Gly His Asn Val Ser Thr
                   85                  90                  95
   Arg Gly Arg Ile Pro Ala Asp Val Ile Asp Ala Tyr His Ala Ala Thr
               100                 105                 110